# NOUVEAU FASCICULE DE BREVET EUROPEEN

(11) **EP 1 046 387 B2**
(45) Date de publication et mention de la décision concernant l'opposition: **02.06.2010**
(45) Mention de la délivrance du brevet: 27.02.2002
(21) Numéro de dépôt: 00400778.7
(22) Date de dépôt: 21.03.2000
(51) Int. Cl.: A61K 8/00, A61Q 1/00, A61Q 5/00, A61Q 19/00

(54) **Composition foisonnée, son procédé de fabrication et son utilisation notamment comme composition cosmétique**
Schaumzusammensetzung, deren Herstellungverfahren sowie deren Anwendung, insbezondere als kosmetische Zusammensetzung
Whipped composition, its production process and its use in particular as a cosmetic composition

(30) Priorité: 20.04.1999 FR 9904968
(43) Date de publication de la demande: 25.10.2000
(73) Titulaire: L'Oréal, 75008 Paris (FR)
(72) Inventeur: Roulier, Véronique, 75010 Paris (FR); Daubige, Thérèse, 77480 Mousseaux les Bray (FR)
(74) Mandataire: Rasson, Catherine

(56) Documents cités:
- EP-A- 0 205 306
- EP-A- 0 835 647
- EP-A- 0 864 317
- EP-A2- 0 422 862
- EP-B1- 0 721 322
- WO-A-97/20626
- WO-A-98/08884
- WO-A2-99//06014
- CH-A5- 674 804
- FR-A- 1 256 438
- US-A- 3 471 624
- US-A- 5 104 643
- US-A- 5 635 469
- COSMETICS & TOILETRIES vol. 100, Juillet 1985, pages 41 - 47
- COSMETICS & TOILETRIES vol. 107, Septembre 1992, pages 131 - 156
- 'Römpps Chemie-Lexikon', 1987 pages 3700 - 3701
- 'Chemicals Engineering', vol. 1, 1995 pages 234 - 280
- 'Arbeiten mit Schaum im Labor' SEIFEN-ÖLE-FETTE-WACHSE ZEITSCHRIFT FÜR DIE WASCHMITTEL-, SEIFEN-, ÖL- UND FETTINDUSTRIE vol. 97, no. 5, Mars 1971, pages 115 - 118
- 'Grundriss der chemischen Technik', 1968 pages 50 - 51
- Anschreiben und Prospekt der Fa. Mondomix, 1996
- Patent Abstracts of Japan, Publ. Nr. 56-079613 & JP 56-079613
- 'International Cosmetic Ingredient Dictionary and Handbook, 7th Ed.', vol. 1, 1997 page 683
- Documentation de HERCULES sur la Natrosol 250
- 'McCutcheon's', vol. 1, 2000 article 'Emulsifiers & Detergents'

## Description

La présente invention se rapporte à une composition foisonnée, contenant un polymère associatif et un tensioactif anionique, et à son utilisation notamment pour le traitement et/ou au nettoyage de la peau humaine, y compris du cuir chevelu, des ongles et/ou des cheveux, et notamment pour le soin des peaux sèches et/ou des lèvres sèches, ainsi qu'au maquillage de la peau.

Les utilisateurs de produits de soin de la peau recherchent de plus en plus des produits agréables à utiliser et ayant une texture originale. Jusqu'à présent, les compositions cosmétiques se présentent le plus souvent sous forme de solutions, de gels ou de crèmes plus ou moins fluides.

Classiquement, les crèmes sont constituées d'une émulsion. Les émulsions comprennent une phase aqueuse et une phase huileuse dispersées l'une dans l'autre. On recherche plus particulièrement les émulsions huile dans eau (H/E) dont la phase externe est la phase aqueuse, car elles apportent plus de fraîcheur à l'application que les émulsions eau-dans-huile (E/H) à phase externe huileuse. Leur toucher et leur application apparaissent moins gras que ceux d'une émulsion E/H. En outre, elles permettent un bon apport d'hydratation, particulièrement utile pour le soin des peaux ou lèvres sèches.

Pour conférer aux émulsions une texture nouvelle, on a cherché à y introduire un gaz, généralement de l'air, pour leur conférer une texture légère et leur donner l'aspect d'une mousse. C'est ce que l'on appelle le foisonnement. Les émulsions foisonnées obtenues sont appréciées pour leur légèreté à l'application. Néanmoins, elles présentent l'inconvénient d'être relativement instables du fait de leur faible densité et donc de décanter après un certain temps de stockage. Le document CH-A-674804 décrit la stabilisation d'une crème cosmétique foisonnée contenant un gaz inerte ou de l'air, par l'ajout d'une solution aqueuse de protéine d'origine animale. Toutefois, on évite l'utilisation de telles protéines dans les produits cosmétiques.

Par ailleurs, le document JP-A-56/079613 décrit des compositions aérées stables exemptes de tensioactif, contenant de 5 à 20% de cires, préférentiellement de cires à haut point de fusion. Les émulsions obtenues sont alors stables mais présentent des propriétés cosmétiques rédhibitoires pour l'utilisateur. En effet, ces compositions contiennent très peu d'eau et manquent donc de fraîcheur lors de l'application sur la peau. En outre, elles contiennent une quantité importante d'humectants tels que la glycérine, ce qui induit une sensation dé collant au toucher. En outre, l'utilisation d'une cire de haut point de fusion en une quantité de 5 % conduit à des textures lourdes très difficiles à appliquer sur la peau.

Il subsiste donc le besoin d'une émulsion ayant l'aspect d'une mousse, tout en contenant une quantité importante d'eau, qui soit fraîche et non collante lors de l'application sur la peau.

La demanderesse a trouvé de manière inattendue que l'utilisation de polymères associatifs en association avec un tensioactif anionique permettait d'obtenir une émulsion foisonnée ayant les propriétés recherchées. Un polymère associatif est un polymère amphiphile, c'est-à-dire un polymère qui comporte au moins une chaîne grasse et des motifs hydrophiles.

Aussi, l'invention a pour objet une composition foisonnée sous forme d'émulsion huile-dans-eau, comprenant dans un milieu physiologiquement acceptable, une phase huileuse dispersée dans une phase aqueuse, caractérisée en ce qu'elle contient de l'air ou un gaz inerte en une quantité d'au moins 30% en volume par rapport au volume total de l'émulsion, pour avoir une densité allant 0,2 à 0,8 (g/cm³) cette densité étant mesurée à une température d'environ 25°C et à la précision atmosphérique, en ce qu'elle contient au moins un polymère amphiphile et au moins un tensioactif anionique et en ce qu'elle comprend au moins un émulsionnant choisi parmi les tensioactifs non ioniques ayant un HLB égal ou supérieur à 9, et les émulsionnants siliconés.

La composition de l'invention présente une densité inférieure à celle d'une émulsion classique et elle reste néanmoins très stable au cours du temps (plusieurs mois à température ambiante). La composition foisonnée de l'invention contient des bulles d'air ou de gaz inerte et a une densité allant de 0,2 à 0,8 (g/cm³) et de préférence de 0,4 à 0,75, cette densité étant mesurée à une température d'environ 25°C et à la pression atmosphérique. Le gaz inerte peut être par exemple l'azote, l'hélium ou l'argon. La quantité d'air ou de gaz inerte nécessaire pour obtenir la densité voulue est d'au moins 30 % en volume et elle peut aller par exemple de 40 à 80 % en volume et de préférence de 50 à 70 % en volume par rapport au volume total de l'émulsion.

La composition de l'invention se différencie des mousses obtenues avec un gaz comprimé, telles que les mousses de rasage par le fait qu'elle reste sous forme de mousse stable dans le temps, contrairement aux mousses de rasage qui se cassent très vite.

La composition de l'invention contient avantageusement un milieu physiologiquement acceptable, c'est-à-dire compatible avec la peau, les yeux et/ou les cheveux et elle peut constituer notamment une composition cosmétique et/ou dermatologique.

Cette composition est une émulsion H/E (huile dans eau) ni grasse ni lourde et, comme la phase externe est la phase aqueuse, elle donne une impression de fraîcheur à l'application sur la peau.

Les polymères susceptibles d'être utilisés dans la présente invention sont des polymères amphiphiles qui comportent au moins une chaîne grasse, donc une partie hydrophobe, et des motifs hydrophiles, donc une partie hydrophile. On les appelle polymères associatifs du fait que le pourcentage et/ou la dimension des groupes hydrophobes sont tels que lesdits groupes hydrophobes sont susceptibles de s'associer, en milieu aqueux, avec d'autres groupes hydrophobes.

La partie hydrophobe peut être en nombre réduit vis-à-vis du reste de la chaîne polymérique et peut se situer latéralement à la chaîne et être répartie de façon aléatoire (copolymèresstatistiques) ou répartie sous forme de séquences ou de greffons (copolymères blocs ou copolymères séquences).

Les polymères utilisables dans la composition de l'invention peuvent être hydrosolubles ou dispersibles dans l'eau pour donner des microgels. Ces polymères sont dits "gonflables" dans l'eau. Les polymères peuvent être de toute nature chimique ; on peut ainsi choisir des polymères d'origine naturelle, éventuellement modifiés ; des polymères radicalaires notamment vinyliques ou acryliques ; des polycondensats, et leurs mélanges. Ces polymères peuvent être ioniques ou non ioniques, et ils sont de préférence anioniques ou non ioniques.

Comme polymères d'origine naturelle, éventuellement modifiés, utilisables dans la composition de l'invention, on peut en particulier citer:
1) les éthers de cellulose possédant des substituants hydrophobes, qui peuvent être des groupes alkyle ayant un nombre de carbone égal ou supérieur à 8. Comme éthers de cellulose de ce type, on peut citer par exemple l'hydroxyéthylcellulose substituée par des groupes hydrophobes, tels que le produit commercialisé sous la dénomination NATROSOL PLUS GRADE 330 par la société Aqualon ;
2) les celluloses cationiques quaternisées modifiées par des groupements comportant au moins une chaîne grasse, tels que des groupes alkyle, arylalkyle, alkylaryle ou leurs mélanges où les groupes alkyle sont de préférence en C₈-C₂₂;
3) les alkylhydroxyéthylcelluloses quaternisées (cationiques) telles que les produits commercialisés sous les dénominations QUATRISOFT LM 200, QUATRISOFT LM-X 529-18-A, QUATRISOFT LM-X 529-18-B (alkyle en C₁₂) et QUATRISOFT LM-X 529-8 (alkyle en C₁₈) par la société Amerchol et les produits commercialisés sous les dénominations CRODACEL QM, CRODACEL QL (alkyle en C₁₂) et CRODACEL QS (alkyle en C₁₈) par la société Croda ;
4) les galactomannanes possédant des substituants hydrophobes, et notamment la gomme de guar substituée hydrophobe. Certains de ces dérivés sont notamment décrits dans le document EP-A-281360 ;
5) les pullulans modifiés par des groupes hydrophobes, en particulier des groupes cholestérol
6) les gélatines modifiées par des groupes hydrophobes, et notamment modifiées par des groupes alkyle en C₆ à C₁₈;
7) les mucopolysaccharides tels que ceux constitués de glycosaminoglycane et d'acide hyaluronique.

Parmi les polycondensats utilisables dans le cadre de l'invention, on peut citer les polyuréthannes associatifs qui sont des copolymères séquencés non ioniques comportant dans la chaîne, à la fois des séquences hydrophiles de nature le plus souvent polyoxyéthylénée et des séquences hydrophobes qui peuvent être des enchaînements aliphatiques seuls ou avec des enchaînements cycloaliphatiques et/ou aromatiques. Les copolymères séquencés résultant peuvent être du type tribloc ou multibloc. Les séquences hydrophobes peuvent donc être à chaque extrémité de la chaîne (copolymères triblocs à séquence centrale polyoxyéthylène) ou réparties à la fois aux extrémités de la chaîne et dans la chaîne (copolymères multiséquencés). Ils peuvent être également en greffons ou en étoile.

Comme polyuréthannes associatifs, on peut citer par exemple les polymères décrits dans l'article de ZEYING MA, J. of Appl. Polymer Sci, vol. 49, 1509-27 (1993) et, parmi les polymères commerciaux, ceux commercialisés sous les dénominations RHEOLATE 205, RHEOLATE 208 et RHEOLATE 204 par la société Rheox. Ces polyuréthannes associatifs sont vendus sous forme pure. On peut aussi utiliser des solutions ou dispersions de ces polymères notamment dans l'eau ou en milieu hydroalcoolique. A titre d'exemples de tels polymères, on peut citer les produits commercialisés sous les dénominations SERAD FX1010 et le SERAD 1035 par la société Hüls, les produits commercialisés sous les dénominations RHEOLATE 255, RHEOLATE 278 et RHEOLATE 244 par la société Rheox. On peut aussi utiliser les produits commercialisés sous les dénominations DW 1206F, DW 1206J, ACRYSOL RM 184, ACRYSOL 44 et ACRYSOL 46 par la société Rohm & Haas. On peut aussi utiliser le produit commercialisé sous la dénomination DW 1206B par la société Rohm & Haas, comportant une chaîne alkyle en C₂₀ et une liaison uréthanne, vendu à 20 % en matière sèche dans l'eau.

Parmi les polymères radicalaires utilisables dans la composition selon l'invention, on peut citer les polymères acryliques anioniques, notamment en dispersion aqueuse, désignés généralement sous le nom de HASE (hydrophobically modified alkalisoluble or swellable emulsion). Ce sont des copolymères acryliques existant sous forme de dispersions dans l'eau à pH acide et qui peuvent se solubiliser dans l'eau par neutralisation complète des groupes anioniques, c'est-à-dire au-delà de pH 8. Certaines de ces dispersions peuvent être partiellement réticulées, ce qui implique que la neutralisation complète ne provoque pas la solubilisation complète des particules de polymère, mais engendre un fort gonflement de ces particules, provoquant également la gélification du milieu.

Ces copolymères, non réticulés ou partiellement réticulés, sont généralement des terpolymères obtenus à partir de (1) un monomère porteur d'un groupe acide carboxylique (acide acrylique ou méthacrylique), (2) un monomère relativement insoluble à l'eau du type acrylate ou méthacrylate en C₁ à C₄, tel que l'acrylate d'éthyle, et (3) un troisième monomère porteur d'un groupe hydrophobe, qui peut être rattaché latéralement à la chaîne principale. Ce groupe hydrophobe peut être un groupe alkyle linéaire ou ramifié comportant au moins 8 atomes de carbone, un groupe cycloalkyle dont le radical alkyle comporte au moins 8 atomes de carbone et/ou un groupe aryle. Le groupe hydrophobe peut être rattaché à la chaîne principale directement par l'intermédiaire d'une liaison éther, ester ou amide, carbamate ou urée. Il peut également être rattaché à la chaîne principale par l'intermédiaire d'une séquence polyoxyéthylénée, elle-même fixée à la chaîne par une liaison éther, ester, amide, carbamate ou urée. Dans ce dernier cas, les groupes latéraux sont généralement de petits greffons à séquence hydrophile et hydrophobe, et les propriétés d'épaississement des milieux aqueux sont plus performantes.

De telles dispersions aqueuses de polymère sont notamment décrites dans SHAY, Surface Coatings International, 1993 (11) 446-453, et dans les documents US-A-4,421,902, US-A-4,423,199, US-A-4,663,385 et US-A-4,384,096. Comme polymères de ce type, on peut par exemple citer les produits décrits ci-après et commercialisés sous les dénominations ACUSOL 823, ACRYSOL 25 et ACUSOL 22 par la société Rohm & Haas.

Parmi les polymères radicalaires utilisables dans la composition selon l'invention, on peut citer :
1) Les copolymères d'acide acrylique ou d'acide méthacrylique avec les N-alkylacrylamides, et en particulier les copolymères acide acrylique / N-alkylacrylamides ayant un groupe alkyle de C₁ à C₂₀, tels que ceux décrits dans l'article de MAGNY et al., Double Liaison, 451, p 52-55 (1993). Ils peuvent être obtenus par copolymérisation directe ou par amidification ultérieure de la chaîne d'acide acrylique. Selon le mode opératoire utilisé, les groupes hydrophobes alkyle peuvent être répartis de façon aléatoire (amidification en solution organique homogène) ou sous forme séquencée (amidification en milieu aqueux où l'amine forme initialement des agrégats de type micellaire).
2) Les copolymères radicalaires anioniques tels que les copolymères obtenus à partir de (a) un monomère à groupe acide carboxylique, par exemple acide acrylique ou acide méthacrylique, et (b) au moins un acrylate, méthacrylate, ester ou amide, porteur de groupes hydrophobes cycloaliphatiques ou aromatiques, tels que des groupes isobornyle ou adamantyle.

On peut encore citer des copolymères avec des monomères perfluorés, en particulier les copolymères avec le (méth)acrylate de perfluorohexyle ; des copolymères entre un monomère porteur d'un groupe acide sulfonique (en particulier acide acrylamido-2-méthyl-2 propane sulfonique, acide styrène sulfonique) et un alkyl-(méth)acrylamide possédant au moins 8 atomes de carbone.
3) Les copolymères acryliques non ioniques, et notamment des copolymères du type acrylamide / N-alkylacrylamide, tels que ceux décrits dans GOODWIN et al., Polymer in Aqueous Media = Performance Through Association, [J.E. Glassed, Adv. Chem. Ser. 223; Am. Chem. Soc., Washington DC, p 365 (1989)].

On peut encore citer les copolymères suivants utilisés seuls ou bien en mélanges :
1) Les copolymères d'anhydride maléique et de monomères comportant au moins une chaîne grasse, tels que les copolymères n-octadécylvinyléther / anhydride maléique, comme le produit commercialisé sous la dénomination GANTREZ AN-8194 par la société ISP.
2) Les copolymères de l'acide crotonique et de monomères comportant au moins une chaîne grasse, tels que les terpolymères acétate de vinyle / acide crotonique / néodécanoate de vinyle, comme le produit commercialisé sous la dénomination RESINE 28-2930 par la société National Starch ; ou les terpolymères acétate de vinyle / acide crotonique / stéarate d'allyle, tels que les produits commercialisés sous les dénominations MEXOMERE PV et PB par la société Chimex.
3) Les polymères d'acide (méth)acrylique, modifiés par des groupes comportant au moins une chaîne grasse, ou les copolymères d'acide (méth)acrylique et de monomères comportant au moins une chaîne grasse, ces monomères étant choisis parmi les monomères hydrophobes à chaîne grasse, les monomères amphiphiles comportant une partie hydrophobe à chaîne grasse et une partie hydrophile.

On peut citer à titre d'exemples de copolymères d'acide (méth)acrylique et de monomères comportant au moins une chaîne grasse :
- les copolymères réticulés d'acide acrylique / acrylate d'alkyle en C₁₀-C₃₀, tels que les produits commercialisés sous les dénominations PEMULEN TR1, PEMULEN TR2, CARBOPOL 1382, CARBOPOL 1342 et CARBOPOL ETD 2020 par la société Goodrich ;
- les copolymères acide (méth)acrylique / acrylate d'éthyle / acrylate d'alkyle, tels que le produit commercialisé sous la dénomination ACUSOL 823 par la société Rohm & Haas et le produit commercialisé sous la dénomination IMPERON R par la société Hoechst ;
- les copolymères réticulés acide acrylique / isodécanoate de vinyle, tels que le produit commercialisé sous la dénomination STABYLEN 30 par la société 3V ;
- les terpolymères acide acrylique / vinylpyrrolidone / méthacrylate de lauryle, tels que les produits commercialisés sous les dénominations ACRYLIDONE LM, ACP-1184, ACP-1194 par la société ISP ;
- les copolymères acide acrylique / (méth)acrylate de lauryle, tels que les produits commercialisés sous les dénominations COATEX SX par la société Coatex ;
- les terpolymères acide (méth)acrylique / acrylate d'alkyle / alkyl-polyéthoxylé allyl éther, tels que les produits commercialisés sous les dénominations RHEOVIS-CR, -CR3, -CR2 et -CRX par la société Allied Colloids ;
- les terpolymères acide méthacrylique / acrylate d'éthyle / stéaryl polyéthoxylé allyl éther, tels que les produits commercialisés sous les dénominations SALCARE-SC90 et -SC80 par la société Allied Colloids (stéaryl polyéthoxylé à 10 moles d'oxyde d'éthylène : en nom CTFA stéareth-10) ;
- les terpolymères acide méthacrylique / acrylate d'éthyle / acrylate de lauryle polyoxyéthyléné, tels que le produit commercialisé sous la dénomination RHEO 2000 par la société Coatex ;
- les terpolymères acide méthacrylique / acrylate d'éthyle / méthacrylate de stéaryle polyoxyéthyléné, tels que les produits commercialisés sous les dénominations ACRYSOL 22, ACRYSOL 25 et DW-1206A par la société Röhm & Haas ;
- les copolymères acide méthacrylique / acrylate d'éthyle / acrylate de nonylphénol polyoxyéthyléné, tels que le produit commercialisé sous la dénomination RHEO 3000 par la société Coatex ;
- les copolymères acide acrylique / monoitaconate de stéaryle polyoxy-éthyléné ou les copolymères acide acrylique / monoitaconate de cétyle polyoxy-éthyléné, tels que les produits commercialisés sous les dénominations 8069-72A et 8069-72B par la société National Starch ;
- les copolymères acide méthacrylique / acrylate de butyle / monomère hydrophobe comportant une chaîne grasse, tels que le produit commercialisé sous la dénomination 8069-146A par la société National Starch ;
- les terpolymères acide acrylique / acrylate d'alkyle en C₁₅ / acrylate de polyéthylène glycol (28 moles d'oxyde d'éthylène), tels que le produit commercialisé sous la dénomination DAPRAL GE 202 par la société Akzo ;
- les sels d'un ester d'acide gras partiel d'un polymère acide acrylique / diméthyléthanolamine, tels que le produit commercialisé sous la dénomination DAPRAL GE 202 DMA par la société Akzo ;
- les copolymères acide acrylique / acrylate monomère amphiphile comportant une chaîne grasse à groupements uréthanne, tels que le produit commercialisé sous la dénomination ADDITOL VXW 1312 par la société Hoechst;
- les polymères acryliques modifiés par des groupes hydrophobes à chaîne grasse, tels que le produit commercialisé sous la dénomination CS-0406 par la société Rohm & Haas.

Les polymères utilisés selon l'invention peuvent être utilisés seuls ou en mélange. Par ailleurs, selon leur nature, ils peuvent être utilisés tels quels, ou sous forme de solutions aqueuses ou de dispersions aqueuses.

La composition selon l'invention comporte avantageusement une quantité de polymère allant de 0,05 à 15 % en poids de matière active, de préférence de 0,1 à 8 % et mieux de 0,2 à 2 % en poids de matière active de polymère par rapport au poids total de la composition.

La composition de l'invention contient au moins un tensioactif anionique. Il s'agit de préférence d'un tensioactif anionique moussant choisi parmi les sulfates, les éthers sulfates et leurs sels. Parmi les sels de sulfates et d'éther sulfates, on choisit préférentiellement les sels de sodium et de triéthanolamine. Comme tensioactif anionique moussant, on peut ainsi utiliser le lauryléthersulfate de sodium et notamment ceux commercialisés sous les dénominations TEXAPON par la société Henkel.

De manière générale, la composition de l'invention contient une quantité de tensioactif anionique allant de 0,5 à 60 % en poids, de préférence de 1 à 30 %, mieux de 1 à 20 % et encore mieux de 2 à 10 % en poids par rapport au poids total de la composition.

La composition selon l'invention contient en outre au moins un émulsionnant choisi parmi les tensioactifs non ioniques ayant un HLB égal ou supérieur à 9, et les émulsionnants siliconés.

Comme émulsionnants, on peut citer par exemple
(1) les tensioactifs non ioniques ayant un HLB supérieur ou égal à 9, tels que les esters d'acide gras et de glycérol oxyéthylénés ; les esters d'acide gras et de sorbitan oxyéthylénés ; les dérivés d'acide gras oxyéthylénés ; les esters d'acide gras et de sucre et notamment les esters gras de sucrose tels que le stéarate de sucrose comme le produit commercialisé sous la dénomination TEGOSOFT PSE 141G par la société Goldschmidt ; les éthers d'alkyl-polyglucoside, et leurs mélanges ;
(2) les émulsionnants siliconés tels que les polydiméthylméthylsiloxanes oxyéthylénés (diméthicone copolyol) comme par exemple celui vendu sous la dénomination "DC2-5695" par la société Dow Corning.

La composition selon l'invention peut comporter par exemple de 0,5 à 30 %, de préférence de 2 à 15 % et mieux de 4 à 10 % en poids d'émulsionnant(s) par rapport au poids total de la composition.

La nature de la phase huileuse de l'émulsion selon l'invention n'est pas critique. La phase huileuse peut ainsi être constituée par tous les corps gras et notamment les huiles, classiquement utilisés dans les domaines cosmétique et dermatologique. La phase huileuse contient au moins une huile, de préférence au moins 1 % en poids d'au moins une huile et mieux au moins 2 % en poids d'au moins une huile, par rapport au poids total de la composition.

Parmi les huiles utilisables dans la composition de l'invention, on peut citer par exemple les huiles végétales telles que l'huile d'abricot ; les huiles minérales comme l'huile de vaseline ; les huiles de synthèse comme l'isohexadécane ; les huiles de silicone volatiles ou non volatiles et les huiles fluorées. Comme huiles de silicone volatile, on peut citer notamment les polydiméthylsiloxanes cycliques ou cyclométhicones qui comportent d'environ 3 à 9 atomes de silicium, et de préférence de 4 à 6 atomes de silicium, telles que le cyclohexadiméthylsiloxane ou cyclohexaméthicone et le cyclopentadiméthylsiloxane ou cyclopentaméthicone. Les autres corps gras susceptibles d'être présents dans la phase huileuse peuvent être par exemple les acides gras, les alcools gras tels que l'alcool cétylique, et les cires.

La composition selon l'invention comporte avantageusement de 1 à 40 % en poids, de préférence de 2 à 30 % en poids et mieux de 5 à 20 % en poids de phase huileuse par rapport au poids total de la composition.

La phase aqueuse de l'émulsion constituant la composition de l'invention peut représenter de 15 à 97,95 % en poids, de préférence de 57 à 93 % et mieux de 75 à 90 % en poids par rapport au poids total de la composition.

De façon connue, la composition de l'invention peut contenir également des adjuvants habituels dans le domaine cosmétique, tels que les actifs, les humectants, les conservateurs, les antioxydants, les agents complexants, les solvants, les parfums, les filtres, les bactéricides, les absorbeurs d'odeur, les matières colorantes (pigments ou colorants solubles) et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

La présente invention se rapporte aussi au procédé de fabrication de la composition selon l'invention. Ce procédé consiste à préparer l'émulsion de manière classique par introduction de la phase huileuse dans la phase aqueuse sous agitation, par exemple dans un appareil de type Moritz, puis à introduire de l'air dans l'émulsion obtenue sous une agitation allant de 500 à 2000 tours/minute, à une température allant de 20°C à 80°C et de préférence de 40°C à 60°C, sous une pression d'entrée d'air allant de 2 à 8 bars (2.10⁵ à 8.10⁵ Pa) et de préférence de 3 à 6 bars (3.10⁵ à 6.10⁵ Pa).

La présente invention a donc aussi pour objet un procédé de fabrication d'une composition foisonnée à base d'une émulsion huile-dans-eau, consistant :
(1) à réaliser de manière classique une émulsion huile dans eau contenant au moins un polymère amphiphile et au moins un tensioactif anionique, par dispersion de la phase huileuse dans la phase aqueuse,
(2) à introduire de l'air dans l'émulsion obtenue sous une agitation allant de 500 à 2000 tours/minute, à une température allant de 20°C à 80°C et sous une pression d'entrée d'air allant de 2 à 8 bars.

Selon un mode préféré de réalisation de l'invention, l'introduction de l'air dans l'émulsion est réalisée dans un appareil de foisonnement comprenant une tête mélangeuse comportant un rotor et un stator, comme par exemple l'appareil "Mondomixer de type Minimondo" fourni par la société Mondomix. L'émulsion est transportée par une pompe dans la tête de foisonnement où l'émulsion et l'air sont simultanément injectés et mélangés de manière homogène grâce à l'action coupante des ergots du rotor et du stator de l'appareil, qui assurent une répartition égale de l'air dans le produit. On règle de manière appropriée la vitesse du rotor de l'appareil, la température de la cuve et des tuyaux ainsi que la pression d'entrée de l'air dans la tête mélangeuse et le débit d'air. La pression de la tête mélangeuse est réglée par un régulateur de pression. Le débit de l'émulsion à la sortie de l'appareil dépend de la fréquence de la pompe en sortie de cuve.

De manière préférée, dans l'appareil de foisonnement, la vitesse d'agitation lors de l'introduction de l'air est de 1000 tours/minute, la température de 50°C et la pression d'entrée d'air de 4 bars.

Dans l'émulsion foisonnée obtenue selon le procédé de l'invention, les bulles d'air ont avantageusement une taille allant de 20 µm à 500 µm, et de préférence allant de 100 µm à 300 µm.

La composition selon l'invention trouve son application dans un grand nombre de traitements, notamment cosmétiques, de la peau, des lèvres et des cheveux, y compris le cuir chevelu, notamment pour le traitement, la protection, le soin, le démaquillage et/ou le nettoyage de la peau, des lèvres et/ou des cheveux, et/ou pour le maquillage de la peau et/ou des lèvres. Elle peut être destinée aussi au traitement des peaux sèches et/ou des lèvres sèches.

La composition selon l'invention peut par exemple être utilisée comme produit de soin, de démaquillage et/ou de nettoyage pour le visage sous forme de crèmes ou de laits ou comme produits de maquillage (peau et lèvres), et par exemple fonds de teint, par incorporation de colorants.

Aussi, l'invention a encore pour objet l'utilisation cosmétique de la composition telle que définie ci-dessus pour le traitement, la protection, le soin, le démaquillage et/ou le nettoyage de la peau, des lèvres et/ou des cheveux, et/ou pour le maquillage de la peau et/ou des lèvres.

L'invention a aussi pour objet un procédé de traitement cosmétique de la peau, y compris du cuir chevelu, des cheveux, et/ou des lèvres, caractérisé par le fait qu'on applique sur la peau, les cheveux et/ou les lèvres, une composition telle que définie ci-dessus.

L'invention a aussi pour objet l'utilisation de la composition telle que définie ci-dessus pour la fabrication d'une composition destinée au soin des peaux sèches et/ou des lèvres sèches.

L'exemple ci-après de compositions selon l'invention est donné à titre d'illustration. Les quantités y sont données en % en poids, sauf mention contraire.

### Exemple : Crème de soin

*Phase huileuse :*
- Huile d'abricot 10 %
- Lauryléthersulfate de sodium 2 %
- Alcool cétylique 2 %
- Stéarate de sucrose (TEGOSOFT PSE 141G) 5 %

*Phase aqueuse :*
- Pemulen TR2 0,4 %
- Conservateurs 1 %
- Eau qsp 100 %

Mode opératoire : on prépare l'émulsion au Moritz de façon classique. Puis, on la met dans la pompe de l'appareil de foisonnement de type Minimondo. De la pompe, elle est transportée vers la tête de foisonnement dans laquelle elle est injectée avec de l'air et mélangée à l'air de manière homogène. Le débit de l'émulsion est de 30 kg/heure, la vitesse du rotor de 1000 tours/minute, la température de la cuve et des tuyaux de 50°C et la pression d'entrée de 4 bars.

On obtient une crème de texture très légère ayant l'aspect d'une mousse fine pouvant être utilisée comme crème de jour.

## Revendications

1. Composition foisonnée sous forme d'émulsion huile-dans-eau, comprenant dans un milieu physiologiquement acceptable, une phase huileuse dispersée dans une phase aqueuse, **caractérisée en ce qu'**elle contient de l'air ou un gaz inerte en une quantité d'au moins 30 % en volume par rapport au volume total de l'émutsion, pour avoir une densité allant de 0,2 à 0,8 (g/cm³) cette densité étant mesurée à une température d'environ 25°C et à la pression atmosphérique, **en ce qu'**elle contient au moins un polymère amphiphile et au moins un tensioactif anionique et **en ce qu'**elle comprend au moins un émulsionnant choisi parmi les tensioactifs non ioniques ayant un HLB égal ou supérieur à 9, et les émulsionnants siliconés.

2. Composition selon la revendication 1, **caractérisée en ce qu'**elle a une densité allant de 0,4 à 0,75.

3. Composition selon la revendication 1 ou 2, **caractérisée en ce que** la quantité d'air ou de gaz inerte va de 40 à 80 % en volume par rapport au volume total de la composition.

4. Composition selon l'une quelconque des revendications précédentes, **caractérisée en ce que** le polymère amphiphile est choisi parmi les polymères d'origine naturelle, éventuellement modifiés, les polymères radicalaires, les polycondensats et leurs mélanges.

5. Composition selon l'une quelconque des revendications précédentes, **caractérisée en ce que** le polymère amphiphile est un polymère d'origine naturelle, choisi parmi les éthers de cellulose possédant des substituants hydrophobes, les celluloses cationiques quaternisées modifiées par des groupements comportant au moins une chaîne grasse, les alkylhydroxyéthylcelluloses quaternisées, les galactomannanes possédant des substituants hydrophobes, les pullulans modifiés par des groupes hydrophobes, les gélatines modifiées par des groupes hydrophobes, les mucopolysaccharides.

6. Composition selon l'une quelconque des revendications 1 à 4, **caractérisée en ce que** le polymère amphiphile est choisi parmi les polyuréthannes associatifs.

7. Composition selon l'une quelconque des revendications 1 à 4, **caractérisée en ce que** le polymère amphiphile est un polymère radicalaire choisi parmi les copolymères d'acide acrylique ou d'acide méthacrylique avec les N-alkylacrylamides, les copolymères obtenus à partir d'un monomère à groupe acide carboxylique, et d'un acrylate, méthacrylate, ester ou amide, porteur de groupes hydrophobes cycloaliphatiques ou aromatiques, les copolymères acryliques non ioniques, les copolymères d'anhydride maléique et de monomères comportant au moins une chaîne grasse, les copolymères de l'acide crotonique et de monomères comportant au moins une chaîne grasse, les polymères d'acide (méth)acrylique, modifiés par des groupes comportant au moins une chaîne grasse, les copolymères d'acide (méth)acrylique et de monomères comportant au moins une chaîne grasse.

8. Composition selon l'une quelconque des revendications 1 à 4, **caractérisée en ce que** le polymère amphiphile est choisi parmi les copolymères réticulés d'acide acrylique / acrylate d'alkyle en C₁₀-C₃₀, les copolymères acide (méth)acrylique / acrylate d'éthyle / acrylate d'alkyle, les copolymères réticulés acide acrylique / isodécanoate de vinyle, les terpolymères acide acrylique / vinylpyrrolidone / méthacrylate de lauryle, les copolymères acide acrylique / (méth)acrylate de lauryle, les terpolymères acide (méth)acrylique / acrylate d'alkyle / alkyl-polyéthoxylé allyl éther, les terpolymères acide méthacrylique / acrylate d'éthyle / stéaryl polyéthoxylé allyl éther, les terpolymères acide méthacrylique / acrylate d'éthyle / acrylate de lauryle polyoxyéthyléné, les terpolymères acide méthacrylique / acrylate d'éthyle / méthacrylate de stéaryle polyoxyéthyléné, les copolymères acide méthacrylique / acrylate d'éthyle / acrylate de nonylphénol polyoxyéthyléné, les copolymères acide acrylique / monoitaconate de stéaryle polyoxy-éthyléné ou les copolymères acide acrylique / monoitaconate de cétyle polyoxy-éthyléné, les terpolymères acide acrylique / acrylate d'alkyle en C₁₅ / acrylate de polyéthylène glycol (28 moles d'oxyde d'éthylène), les copolymères acide acrylique / acrylate / monomère amphiphile comportant une chaîne grasse à groupements uréthanne, les polymères acryliques modifiés par des groupes hydrophobes à chaîne grasse, et leurs mélanges.

9. Composition selon l'une quelconque des revendications précédentes, **caractérisée en ce qu'**elle contient une quantité de polymère(s) amphiphile(s) allant de 0,05 à 15 % en poids de matière active, par rapport au poids total de la composition.

10. Composition selon l'une quelconque des revendications précédentes, **caractérisée en ce que** le tensioactif anionique est choisi parmi les sulfates, les éthers sulfates et leurs sels.

11. Composition selon l'une quelconque des revendications précédentes, **caractérisée en ce que** le tensioactif anionique est le lauryléthersulfate de sodium.

12. Composition selon l'une quelconque des revendications précédentes, **caractérisée en ce qu'**elle contient une quantité de tensioactif(s) anionique(s) allant de 1 à 20 % en poids par rapport au poids total de la composition.

13. Composition selon l'une quelconque des revendications précédentes, **caractérisée en ce qu'**elle contient une quantité d'émulsionnant(s) allant de 0,5 à 30 % en poids par rapport au poids total de la composition.

14. Composition selon l'une quelconque des revendications précédentes, **caractérisée en ce que** la phase huileuse représente de 1 à 40 % en poids par rapport au poids total de l'émulsion.

15. Procédé de fabrication d'une composition foisonnée à base d'une émulsion huile-dans-eau selon l'une quelconque des revendications précédentes, consistant :
(1) à réaliser de manière classique une émulsion huile dans eau contenant un polymère amphiphile et un tensioactif anionique, par dispersion de la phase huileuse dans la phase aqueuse,
(2) à introduire de l'air dans l'émulsion obtenue sous une agitation allant de 500 à 2000 tours/minute, à une température allant de 20°C à 80°C et sous une pression d'entrée d'air allant de 2 à 8 bars (2.10⁵ à 8.10⁵ Pa).

16. Procédé selon la revendication 15, **caractérisé en ce que** l'introduction d'air est réalisée dans un appareil de foisonnement.

17. Procédé selon la revendication 15 ou 16, **caractérisé en ce que** la composition est telle que définie, selon l'une quelconque des revendications 1 à 14.

18. Composition foisonnée obtenue par le procédé selon l'une quelconques des revendications 15 à 17.

19. Utilisation cosmétique de la composition selon l'une quelconque des revendications 1 à 14 et 18, pour le traitement, la protection, le soin, le démaquillage et/ou le nettoyage de la peau, des lèvres et/ou des cheveux, et/ou pour le maquillage de la peau et/ou des lèvres.

20. Procédé de traitement cosmétique de la peau, y compris du cuir chevelu, des cheveux, et/ou des lèvres, **caractérisé par le fait qu'**on applique sur la peau, les cheveux et/ou les lèvres, une composition selon l'une quelconque des revendications 1 à 14 et 18.

21. Utilisation de la composition selon l'une quelconque des revendications 1 à 14 et 18, pour la fabrication d'une composition destinée au soin des peaux sèches et/ou des lèvres sèches.

## Claims

1. Expanded composition in the form of an oil-in-water emulsion comprising, in a physiologically acceptable medium, an oily phase dispersed in an aqueous phase, **characterized in that** it comprises air or an inert gas in an amount of at least 30% by volume with respect to the total volume of the emulsion, in order to have a density ranging from 0.2 to 0.8 (g/cm³), this density being measured at a temperature of approximately 25°C and at atmospheric pressure, **in that** it comprises at least one amphiphilic polymer and at least one anionic surfactant and **in that** it comprises at least one emulsifier chosen from nonionic surfactants having an HLB of equal to or greater than 9 and silicone emulsifiers.

2. Composition according to Claim 1, **characterized in that** it has a density ranging from 0.4 to 0.75.

3. Composition according to Claim 1 or 2, **characterized in that** the amount of air or of inert gas ranges from 40 to 80% by volume with respect to the total volume of the composition.

4. Composition according to any one of the preceding claims, **characterized in that** the amphiphilic polymer is chosen from optionally modified polymers of natural origin, radical polymers, polycondensates and their mixtures.

5. Composition according to any one of the preceding claims, **characterized in that** the amphiphilic polymer is a polymer of natural origin chosen from cellulose ethers possessing hydrophobic substituents, quaternized cationic celluloses modified by groups comprising at least one fatty chain, quaternized alkylhydroxyethylcelluloses, galactomannans possessing hydrophobic substituents, pullulans modified by hydrophobic groups, gelatins modified by hydrophobic groups, or mucopolysaccharides.

6. Composition according to any one of Claims 1 to 4, **characterized in that** the amphiphilic polymer is chosen from associative polyurethanes.

7. Composition according to any one of Claims 1 to 4, **characterized in that** the amphiphilic polymer is a radical polymer chosen from copolymers of acrylic acid or of methacrylic acid with N-alkyl-acrylamides, copolymers obtained from a monomer comprising a carboxylic acid group and from an acrylate, methacrylate, ester or amide carrying hydrophobic cycloaliphatic or aromatic groups, nonionic acrylic copolymers, copolymers of maleic anhydride and of monomers comprising at least one fatty chain, copolymers of crotonic acid and of monomers comprising at least one fatty chain, (meth)acrylic acid polymers modified by groups comprising at least one fatty chain, or copolymers of (meth)acrylic acid and of monomers comprising at least one fatty chain.

8. Composition according to any one of Claims 1 to 4, **characterized in that** the amphiphilic polymer is chosen from acrylic acid/C₁₀-C₃₀ alkyl acrylate crosslinked copolymers, (meth)acrylic acid/ethyl acrylate/alkyl acrylate copolymers, acrylic acid/vinyl isodecanoate crosslinked copolymers, acrylic acid/vinylpyrrolidone/lauryl methacrylate terpolymers, acrylic acid/lauryl (meth)acrylate copolymers, (meth)acrylic acid/alkyl acrylate/polyethoxylated alkyl allyl ether terpolymers, methacrylic acid/ethyl acrylate/polyethoxylated stearyl allyl ether terpolymers, methacrylic acid/ethyl acrylate/polyoxyethylenated lauryl acrylate terpolymers, methacrylic acid/ethyl acrylate/polyoxyethylenated stearyl methacrylate terpolymers, methacrylic acid/ethyl acrylate/polyoxyethylenated nonylphenol acrylate copolymers, acrylic acid/polyoxyethylenated stearyl monoitaconate copolymers or acrylic acid/polyoxyethylenated cetyl monoitaconate copolymers, acrylic acid/C₁₅ alkyl acrylate/polyethylene glycol acrylate (28 mol of ethylene oxide) terpolymers, acrylic acid/acrylate/amphiphilic monomer comprising a fatty chain comprising urethane groups copolymers, acrylic polymers modified by hydrophobic groups comprising a fatty chain, and their mixtures.

9. Composition according to any one of the preceding claims, **characterized in that** it comprises an amount of amphiphilic polymer(s) ranging from 0.05 to 15% by weight of active material with respect to the total weight of the composition.

10. Composition according to any one of the preceding claims, **characterized in that** the anionic surfactant is chosen from sulphates, ether sulphates and their salts.

11. Composition according to any one of the preceding claims, **characterized in that** the anionic surfactant is sodium lauryl ether sulphate.

12. Composition according to any one of the preceding claims, **characterized in that** it comprises an amount of anionic surfactant (s) ranging from 1 to 20% by weight with respect to the total weight of the composition.

13. Composition according to any one of the preceding claims, **characterized in that** it comprises an amount of emulsifier(s) ranging from 0.5 to 30% by weight with respect to the total weight of the composition.

14. Composition according to any one of the preceding claims, **characterized in that** the oily phase represents from 1 to 40% by weight with respect to the total weight of the emulsion.

15. Process for the manufacture of an expanded composition based on an oil-in-water emulsion according to any one of the preceding claims, which consists:
(1) in preparing an oil-in-water emulsion comprising an amphiphilic polymer and an anionic surfactant in a conventional way by dispersing the oily phase in the aqueous phase,
(2) in introducing air into the emulsion obtained with stirring ranging from 500 to 2000 revolutions/minute, at a temperature ranging from 20°C to 80°C and under an air inlet pressure ranging from 2 to 8 bar (2 × 10⁵ to 8 × 10⁵ Pa).

16. Process according to Claim 15, **characterized in that** the introduction of air is carried out in an expansion device.

17. Process according to Claim 15 or 16, **characterized in that** the composition is as defined according to any one of Claims 1 to 14.

18. Expanded composition obtained by the process according to any one of Claims 15 to 17.

19. Cosmetic use of the composition according to any one of Claims 1 to 14 and 18 for treating, protecting, caring for, removing make-up from and/or cleansing the skin, lips and/or hair and/or for making up the skin and/or lips.

20. Process for the cosmetic treatment of the skin, including the scalp, hair and/or lips, **characterized in that** a composition according to any one of Claims 1 to 14 and 18 is applied to the skin, hair and/or lips.

21. Use of the composition according to any one of Claims 1 to 14 and 18 in the manufacture of a composition intended for caring for dry skin and/or dry lips.

## Patentansprüche

1. Aufgeschäumte Zusammensetzung in Form einer Öl-in-Wasser-Emulsion, die in einem physiologisch akzeptablen Medium eine in einer wäßrigen Phase dispergierte Ölphase enthält, **dadurch gekennzeichnet, dass** sie Luft oder ein inertes Gas in einer Menge von mindestens 30 Vol.-%, bezogen auf das Gesamtvolumen der Emulsion enthält, so dass eine Dichte im Bereich von 0,2 bis 0,8 (g/cm³) bei einer Temperatur erhalten wird, wobei diese Dichte bei einer Temperatur von 25 °C und bei Atmosphärendruck gemessen wird, und dass sie mindestens ein amphiphiles Polymer und mindestens einen anionischen grenzflächenaktiven Stoff enthält und dass sie mindestens einen Emulgator enthält, der unter den nichtionischen grenzflächenaktiven Stoffe, die einen HLB-Wert von 9 oder darüber aufweisen, und den siliconhaltigen Emulgatoren ausgewählt wird.

2. Zusammensetzung nach Anspruch 1, **dadurch gekennzeichnet, dass** sie eine Dichte von 0,4 bis 0,75 aufweist.

3. Zusammensetzung nach Anspruch 1 oder 2, **dadurch gekennzeichnet, dass** die Menge an Luft oder inertem Gas im Bereich von 40 bis 80 Vol.-%, bezogen auf das Gesamtvolumen der Zusammensetzung, liegt.

4. Zusammensetzung nach einem der vorhergehenden Ansprüche, **dadurch gekennzeichnet, dass** das amphiphile Polymer unter den Polymeren natürlicher Herkunft, die gegebenenfalls modifiziert sind, den radikalischen Polymeren, den Polykondensaten und ihren Gemischen ausgewählt ist.

5. Zusammensetzung nach einem der vorhergehenden Ansprüche, **dadurch gekennzeichnet, dass** das amphiphile Polymer ein Polymer natürlicher Herkunft ist, das unter den Celluloseethern, die hydrophobe Substituenten aufweisen, den quaternisierten kationischen Cellulosen, die mit Gruppen modifiziert sind, die mindestens eine Fettkette enthalten, den quaternisierten Alkylhydroxyethylcellulosen, den Galactomannanen, die hydrophobe Substituenten aufweisen, den Pullulanen, die mit hydrophoben Gruppen modifiziert sind, den Gelatinen, die mit hydrophoben Gruppen modifiziert sind, den Mucopolysacchariden ausgewählt ist.

6. Zusammensetzung nach einem der Ansprüche 1 bis 4, **dadurch gekennzeichnet, dass** das amphiphile Polymer unter den assoziativen Polyurethanen ausgewählt ist.

7. Zusammensetzung nach einem der Ansprüche 1 bis 4, **dadurch gekennzeichnet, dass** das amphiphile Polymer ein radikalisches Polymer ist, das ausgewählt ist unter den Copolymeren aus Acrylsäure oder Methacrylsäure mit N-Acrylamiden, den Copolymeren, die aus einem Monomer mit Carbonsäuregruppe und einem Acrylat, Methacrylat, Ester oder Amid, das cycloaliphatische oder aromatische hydrophobe Gruppen trägt, erhalten werden, den nichtionischen Acryl-Copolymeren, den Copolymeren aus Maleinsäureanhydrid und Monomeren, die mindestens eine Fettkette aufweisen, den Copolymeren aus Crotonsäure und Monomeren, die mindestens eine Fettkette aufweisen, den Polymeren der (Meth)acrylsäure, die mit Gruppen modifiziert sind, die mindestens eine Fettkette aufweisen, den Copolymeren aus (Meth)acrylsäure und Monomeren, die mindestens eine Fettkette aufweisen.

8. Zusammensetzung nach einem der Ansprüche 1 bis 4, **dadurch gekennzeichnet, dass** das amphiphile Polymer ausgewählt ist unter den vernetzten Acrylsäure/C₁₀-C₃₀-Alkylacrylat-Copolymeren, den (Meth)acrylsäure/Ethylacrylat/Alkylacrylat-Copolymeren, den vernetzten Acrylsäure/Vinylisodecanoat-Copolymeren, den Acrylsäure/Vinylpyrrolidon/Laurylmethacrylat-Copolymeren, den Acrylsäure/Lauryl(meth)acrylat-Terpolymeren, den Terpolymeren aus (Meth)acrylsäure, Alkylacrylat und Allylether mit polyethoxyliertem Alkyl, den Terpolymeren aus Methacrylsäure, Ethylacrylat und Allylether mit polyethoxyliertem Stearylalkohol, den Terpolymeren aus Methacrylsäure, Ethylacrylat und einem Ester aus Acrylsäure und polyethoxyliertem Laurylalkohol, den Terpolymeren aus Methacrylsäure, Ethylacrylat und einem Ester aus Methacrylsäure und polyethoxyliertem Stearylalkohol, den Copolymeren aus Methacrylsäure, Ethylacrylat und einem Ester aus Acrylsäure und polyethoxyliertem Nonylphenol, den Copolymeren aus Acrylsäure und einem Monoester aus Itaconsäure und polyethoxyliertem Stearylalkohol, den Copolymeren aus Acrylsäure und einem Monoester aus Itaconsäure und polyethoxyliertem Cetylalkohol, den Terpolymeren aus Acrylsäure, C₁₅-Alkylacrylat und Polyethylenglykolacrylat (28 mol Ethylenoxid), den Copolymeren aus Acrylsäure, Acrylat und amphiphilem Monomer, das eine Fettkette mit Urethangruppen aufweist, den Acryl-Polymeren, die mit hydrophoben Gruppen mit Fettkette modifiziert sind, und ihren Gemischen.

9. Zusammensetzung nach einem der vorhergehenden Ansprüche, **dadurch gekennzeichnet, dass** sie eine Menge an amphiphilem Polymer oder amphiphilen Polymeren enthält, die im Bereich von 0,05 bis 15 Gew.-% Wirkstoff, bezogen auf das Gesamtgewicht der Zusammensetzung, liegt.

10. Zusammensetzung nach einem der vorhergehenden Ansprüche, **dadurch gekennzeichnet, dass** der anionische grenzflächenaktive Stoff unter den Sulfaten, den Ethersulfaten und ihren Salzen ausgewählt ist.

11. Zusammensetzung nach einem der vorhergehenden Ansprüche, **dadurch gekennzeichnet, dass** es sich bei dem anionischen grenzflächenaktiven Stoff um Natriumlaurylethersulfat handelt.

12. Zusammensetzung nach einem der vorhergehenden Ansprüche, **dadurch gekennzeichnet, dass** sie eine Menge an anionischem grenzflächenaktivem Stoff oder anionischen grenzflächenaktiven Stoffen enthält, die im Bereich von 1 bis 20 Gew.-%, bezogen auf das Gesamtgewicht der Zusammensetzung, liegt.

13. Zusammensetzung nach einem der vorhergehenden Ansprüche, **dadurch gekennzeichnet, dass** sie eine Menge an einem Emulgator oder mehreren Emulgatoren enthält, die im Bereich von 0,5 bis 30 Gew.-%, bezogen auf das Gesamtgewicht der Zusammensetzung, liegt.

14. Zusammensetzung nach einem der vorhergehenden Ansprüche, **dadurch gekennzeichnet, dass** der Anteil der Ölphase 1 bis 40 Gew.-% beträgt, bezogen auf das Gesamtgewicht der Emulsion.

15. Verfahren zur Herstellung einer aufgeschäumten Zusammensetzung auf der Basis einer Öl-in-Wasser-Emulsion nach einem der vorhergehenden Ansprüche, das folgende Schritte umfaßt:
(1) Herstellen in klassischer Weise einer Öl-in-Wasser-Emulsion, die ein amphiphiles Polymer und einen anionischen grenzflächenaktiven Stoff enthält, durch Dispergieren der Ölphase in der wäßrigen Phase,
(2) Einbringen von Luft in die erhaltene Emulsion unter Rühren mit einer Rührgeschwindigkeit von 500 bis 2000 U/min bei einer Temperatur im Bereich von 20 bis 80 °C und bei einem Einspeisedruck der Luft von 2 bis 8 bar (2·10⁵ bis 8·10⁵ Pa).

16. Verfahren nach Anspruch 15, **dadurch gekennzeichnet, dass** das Einbringen der Luft in einem Expandiergerät durchgeführt wird.

17. Verfahren nach Anspruch 15 oder 16, **dadurch gekennzeichnet, dass** die Zusammensetzung so wie in einem der Ansprüche 1 bis 14 definiert ist.

18. Aufgeschäumte Zusammensetzung, die nach dem Verfahren nach einem der Ansprüche 15 bis 17 hergestellt ist.

19. Kosmetische Verwendung der Zusammensetzung nach einem der Ansprüche 1 bis 14 und 18 für die Behandlung, den Schutz, die Pflege, das Abschminken und/oder die Reinigung der Haut, der Lippen und/oder der Haare und/oder zum Schminken der Haut und/oder der Lippen.

20. Verfahren zur kosmetischen Behandlung der Haut einschließlich der Kopfhaut, der Haare und/oder der Lippen, **dadurch gekennzeichnet, dass** auf die Haut, die Haare und/oder die Lippen eine Zusammensetzung nach einem der Ansprüche 1 bis 14 und 18 aufgetragen wird.

21. Verwendung der Zusammensetzung nach einem der Ansprüche 1 bis 14 und 18 für die Herstellung einer Zusammensetzung, die für die Pflege von trockener Haut und/oder trockenen Lippen vorgesehen ist.
